Europäisches Patentamt

(19) European Patent Office

Office européen des brevets

(11) Publication number : **0 359 746 B1**

(12) **EUROPEAN PATENT SPECIFICATION**

(45) Date of publication of patent specification :
03.06.92 Bulletin 92/23

(51) Int. Cl.⁵ : **A61K 9/22**

(21) Application number : **88903051.6**

(22) Date of filing : **23.03.88**

(86) International application number :
**PCT/US88/00696**

(87) International publication number :
**WO 88/07366 06.10.88 Gazette 88/22**

(54) **USE OF VINYL ALCOHOL HOMOPOLYMERS AND COPOLYMERS FOR TABLETING ACTIVE MATERIALS.**

(30) Priority : **25.03.87 US 29996**

(43) Date of publication of application :
**28.03.90 Bulletin 90/13**

(45) Publication of the grant of the patent :
**03.06.92 Bulletin 92/23**

(84) Designated Contracting States :
**BE CH DE FR GB IT LI NL**

(56) References cited :
**US-A- 3 080 346
US-A- 4 547 359
US-A- 4 695 467
CHEMICAL ABSTRACTS, vol. 103, no. 12, 23rd
September 1985, page 314, abstract no.
92739u, Columbus, Ohio, US; R. GURNY etal.:
"New observations on the mechanism of re-
lease of a solute from hydrophilic polymers",
& EXPO. - CONGR. INT. TECHNOL.PHARM.,
3rd 1983, 2, 97-105
IL FARMACO. EDIZIONE PRATICS, vol. 38, no.
10, October 1983, pages 361-368, Pavia, IT; U.
CONTE et al.: "The compactabilityof polymers
for drug delivery systems"
Pharmazie, vol. 38, no. 7 (1983) W. Suess, "On
the production of protracted release moldings
based on hydrophilic matrixforms by means of
polyvinyl alcohol" pages 476-478**

(73) Proprietor : **E.I. DU PONT DE NEMOURS AND
COMPANY
1007 Market Street
Wilmington Delaware 19898 (US)**

(72) Inventor : **BATEMAN, Linda, R.
1608 Turkey Run Road
Wilmington, DE 19803 (US)**
Inventor : **COFFIN-BEACH, David, P.
78 West Bayview Avenue
Lindenhurst, NY 11757 (US)**
Inventor : **DI LUCCIO, Robert, C.
9 Carsdale Court
Wilmington, DE 19808 (US)**
Inventor : **STEWART, Clare, A., Jr.
407 Brentwood Drive
Wilmington, DE 19803 (US)**
Inventor : **VISIOLI, Donna, L.
38 Austin Circle
Lower Swyneed, PA 19002 (US)**

(74) Representative : **de Bruijn, Leendert C. et al
Nederlandsch Octrooibureau
Scheveningseweg 82 P.O. Box 29720
NL-2502 LS 's-Gravenhage (NL)**

Note : Within nine months from the publication of the mention of the grant of the European patent, any
person may give notice to the European Patent Office of opposition to the European patent granted.
Notice of opposition shall be filed in a written reasoned statement. It shall not be deemed to have been
filed until the opposition fee has been paid (Art. 99(1) European patent convention).

EP 0 359 746 B1

## Description

FIELD OF THE INVENTION

This invention relates to the manufacture of compressed tablets for such uses as oral drug delivery for medical or veterinary use, delivery of pesticides and herbicides to the application sites, and dispensing measured quantities of personal care products, more particularly the manufacture of tablets having a range of the active ingredient release characteristics from instant to prolonged, utilizing homopolymer and copolymers of vinyl alcohol as binder.

BACKGROUND OF THE INVENTION

Compressed tablets for such uses as oral drug delivery are prepared by three methods--wet granulation, dry granulation and direct compression. Each method involves mixing powdered drug substance with a powdered binder material and compressing the mixture in a tableting machine. In the wet granulation method the mixture is granulated before compression by wetting with a solution of the binder while stirring, wet screening, drying and dry screening. In the dry granulation method the mixture is granulated before compression by dry-compacting then dry screening. In the direct compression method the powder mixture is compressed to form the tablet without intermediate granulation.

Binders which are used commercially in the manufacture of compressed tablets include lactose, hydroxypropylmethylcellulose, microcrystalline cellulose, acacia mucilage, tragacanth mucilage, starch mucilage, alginates, sugar and polyvinyl-pyrrolidone. Commercial prolonged-action tablets include tablets with slow-release cores, tableted mixed-release granules, multiple-layer tablets, drug-filled porous inert plastic pellets, and tablets of drug-ion exchange resin complexes. These are relatively complex formulations compared to conventional quick-release tablets and are thus more costly to manufacture. Il Farmaco, Edizione Pratics, Vol. 38 (1983), no. 10, pages 361-368 features the relation between packing and compacting properties and the selection of materials. In particular it was concluded that the greater the packing fraction of powder bed, that pouring the compaction properties and that the polymeric materials showing compression for -displacement cycles not well balanced between their various faces do not yield cohesive compacts. The document mentions the use of polyvinylalcohol in the preparation of drug delivery system (direct compression tablets). U.S. Patents 3,870,790, 4,226,849 and 4,389,393 disclose sustained-release tablets made by conventional tablet-making methods using certain hydroxypropylmethylcelluloses (HPMC's) as binders. Although the HPMC's are being used commercially, their bulk flow characteristics and compressibility are rather poor; typically granulation prior to compression and high compression pressures are required, which may affect process and product reproducibility and add to the cost of manufacture.

Suess, Pharmazie, 38, No. 7, 476-8 (1983) describes preparation of compressed tablets by the direct compression method using polyvinyl alcohol (PVA) and drug substance. Inclusion of various quantities of magnesium stearate to provide delayed release is described. Protracted release was correlated to addition of other excipients, i.e., potato starch and magnesium stearate, rather than inclusion of PVA. Physical and chemical characteristics of the PVA used are not described. However, most commercially available PVA, because of its method of manufacture, is only partially hydrolyzed and consists of non-spheroidal particles with size distribution such that more than 50% of the material is retained on a 60 mesh (0.246 mm opening) Tyler screen. Such PVA particles have erratic flow and compressibility characteristics.

PVA has also been used to prepare controlled release drug formulations other than compressed tablets. For example, Korsmeyer et al., Journal of Membrane Science, 9, 211-227 (1981) describes preparation of PVA gels containing drug substance for controlled drug release. The gels were made by swelling PVA in water, adding drug substance and cross-linking with glutaraldehyde. The PVA used by Korsmeyer et al. was Elvanol® grade 85-82 from E. I. du Pont de Nemours and Company, Wilmington, Delaware. Korsmeyer et al. describes this PVA as an amorphous and atactic polymer with number average molecular weight $\overline{Mn}$=52,800, poly-dispersity index $\overline{Mw}/\overline{Mn}$=2.14, and degree of hydrolysis = 99.8%. Keith et al. U.S. Patent 4,291,014 discloses dissolving PVA and polyvinylpyrrolidone in water, adding a drug substance and pouring the mixture into forms to provide a diffusion matrix for controlled drug release. Numerous patents and published applications disclose controlled release oral formulations comprising drug substance in powder, pill, tablet or capsule form having a polymer coating comprising PVA. Examples of such patents and applications are PCT publication WO 85/03436, PCT publication WO 85/03437, EPO publication 0 063 014; EPO publication 0 076 428; U.S. Patent 4,205,060, U.S. Patent 4,432,965; and U.K. publication GB 2042892A. German published application 30 42 916 discloses a tablet made by applying a drug to the surface of a film of polyvinyl alcohol or a fiber fleece bonded with polyvinyl alcohol.

Compressed tablets containing other active ingredients such as, for example, pesticides or herbicides; fertlizers, especially for use on house plants; personal care products such as, for example, denture cleaners; household preparations such as, for example, bathroom cleaners, have been used to a lesser degree, either because of the high cost of tableting those materials relative to their value in use or because of the difficulty in controlling the rate of release of the active ingredient to the desired extent.

Tableting agricultural formulations such as, e.g., pesticides or herbicides offers many advantages over the usual dusts, powders, and granules. For example, tableting does not adversely affect heat-labile materials since it requires no heat; it prevents or reduces health and environment hazards encountered in the case of dusts and powders (and even granules); and it permits release of the active ingredient at a controlled rate.

Herbicides often are added in fertilizer solutions. Currently, they are supplied as dry flowable powders, which must be measured by the farm operator before adding to the fertilizer spray tank. This causes waste and exposes the operator to a biologically active material. Herbicides used for crop dusting need to have increased density and very specific geometry for accurate application.

Slugs are a serious pest to crops in many parts of the world. Current slug baits, consisting of wheat flour, pesticide, and paraffin wax binder, break up rapidly on exposure to damp earth and rain. A longer release slug bait would be very desirable.

Tableting solves all the above problems of the current agricultural formulations.

Industrial chemicals such as, e.g., oxidizing agents used for the purification and sanitation of spas and swimming pools normally are rapidly released into water shortly after their application. It would be desirable to control the rate of their release in order to minimize the frequency of their application and to avoid wide fluctuations in concentration.

Personal care products such as, e.g., denture cleaners and contact lens cleaners in tablet form permit the user to employ a predetermined effective amount of the active ingredient.

Veterinary chemicals in tablet form permit controlled release of nutritional supplements for animals such as dairy and beef cattle.

## SUMMARY OF THE INVENTION

We have found that pharmaceutically elegant and esthetically appealing compressed tablets having a range of active ingredient release characteristics, from substantially instant to prolonged release, can be made by the wet granulation, dry granulation or direct compression method, using a mixture of active ingredient and binder in powder form "The binder being characterized by consisting essentially of at least one polymer selected from the group consisting of polyvinyl alcohol and copolymers of vinyl alcohol with up to 10 mole percent methyl acrylate or methyl methacrylate, the polymer having a number average molecular weight $M_n$ in the range of 25,000 to 100,000; polydispersity ($M_w/M_n$) in the range of 1.4 to 2.6, where $M_w$ is weight average molecular weight; viscosity in 4% aqueous solution in the range of 10 to 70 mPa.s; a surface area in the range of 0.6 to 6.0 $m^2/g$; percent hydrolysis of at least 98%; the polymer being in the form of spheroidal particles arranged in spheroidal clusters, with a size distribution such that less than 5% of the polymer is retained on a 20 mesh (0.833 mm) screen; less than 50% is retained on an 80 mesh (0.175 mm) screen; and more than 70% is retained on a 200 mesh (0.074 mm) screen. Preferably, more than 90% is retained on a 400 mesh (0.038 mm) screen. Vinyl alcohol polymers having particles of the described shape and size are referred to herein as granular polymers.

By pharmaceutically elegant and esthetically appealing tablets we mean tablets which have suitable hardness and coherence, excellent appearance and excellent uniformity of weight, physical dimensions and hardness. The vinyl alcohol polymers permit formulation of tablets which have active ingredient release characteristics which are independent of processing changes and which are predictable (in vitro) using classical diffusion controlled modeling techniques. They also permit formulation of tablets which have the desirable characteristic of floating in water, and continuing to float when thoroughly wetted.

The invention further provides compressed tablets for active ingredient delivery. Said tablets are characterized in that they consist essentially of active ingredient and binder consisting essentially of 30 to 100% of polymers selected from the group consisting of copolymers of vinyl alcohol with 1 to 10 mole per cent methyl acrylate, the polymer having: a number average molecular weight in the range of 25,000 to 100,000; polydispersity in the range of 1.4 to 2.6; viscosity in 4% aqueous solution in the range of 10 to 70 mPa.s; a surface area in the range of 0.6 to 6.0 $m^2/g$; percent hydrolysis of at least 98%; the polymer being in the form of spheroidal particles arranged in spheroidal clusters, with a size distribution such that less than 5% of the polymer is retained on a 20 mesh (0.833 mm) screen, less than 50% is retained on an 80 mesh (0.175 mm) screen, and more than 70% is retained on a 200 mesh (0.074 mm) screen and 0 to 70% of at least one member of the group consisting of polyvinyl alcohol, hydroxypropyl methyl cellulose, microcrystalline cellulose, lactose, acacia mucilage, tragacanth mucilage, starch mucilage, alginates, sugar, and polyvinylpyrrolidone.

The invention also provides compressed tablets for immediate oral drug delivery consisting essentially of drug substance and binder. Said compressed tablets are characterized in that the binder consists essentially of at least one polymer selected from the group consisting of polyvinyl alcohol and copolymers of vinyl alcohol with up to 10 mole % of methyl methacrylate or methyl methacrylate, the polymer having: a number average molecular weight in the range of 25,000 to 100,000; polydispersity in the range of 1.4 to 2.6; viscosity in 4% aqueous solution in the range of 10 to 70 mPa.s; a surface area in the range of 0.6 to 6.0 $m^2/g$; percent hydrolysis of at least 98%; the polymer being in the form of spheroidal particles arranged in spheroidal clusters, with a size distribution such that less than 5% of the polymer is retained on a 20 mesh (0.833 mm) screen, less than 50% is retained on an 80 mesh (0.175 mm) screen, and more than 70% is retained on a 200 mesh (0.074 mm) screen, said polymer being at least partially crystalline, whereby the tablet disintegrates quickly when placed in dilute acid.

Throughout this specification and the claims, parts and percentages are by weight, unless otherwise specified. Vinyl alcohol polymers are made by hydrolysis or alcoholysis of vinyl acetate polymers. Percent hydrolysis refers to the mole percent hydrolysis or alcoholysis of acetate groups, dry basis. Also, throughout this specification and the claims, viscosity figures are in mPa.s for 4% aqueous solutions measured by the Hoeppler falling ball method; surface areas are in $m^2/g$ measured by the BET method; and sieve sizes refer to the Tyler sieve series.

Granular vinyl alcohol polymers having the characteristics described exhibit excellent flow and compression characteristics, and therefore provide significant processing and cost advantages in the manufacture of compressed tablets. In a standard flowability test using a Hanson Flowdex®, we have found that a polymer useful in this invention is capable of flowing spontaneously through an orifice of 10 millimeter diameter. Using a standard Strong-Cobb instrument for testing tablet hardness, we have found that the vinyl alcohol polymers described, when compressed without other ingredients, using a compression pressure of $12.3 \times 10^6$ kg/$m^2$ ($12.46 \times 10^7$Pa), provide tablets having a hardness of at least 30 Strong-Cobb units (SCU). Microcrystalline cellulose and hydroxypropylmethylcellulose used commercially as tablet binder and non-granular vinyl alcohol polymers do not exhibit the desirable flowability and compressibility characteristics of the granular vinyl alcohol polymers used in this invention. Although wet granulation can be used in the method of this invention, dry granulation or direct compression is satisfactory. Direct compression is usually adequate and is preferred.

We have found that vinyl alcohol polymers which are at least partially crystalline, as a result of being heat-treated after alcoholysis, provide tablets which give immediate active ingredient release, e.g., nearly all of the active ingredient being released within 30 minutes or less. Such tablets are novel, and constitute an embodiment of this invention.

Vinyl alcohol polymers which are not heat-treated and are substantially amorphous-provide tablets which give prolonged active ingedient release, e.g., 24 hours or longer to release the active ingredient. By blending crystalling and amorphous polymers in various ratios, tablets having a range of active ingredient release characteristics can be provided.

## DESCRIPTION OF THE DRAWINGS

FIG. 1 is a wide angle x-ray diffraction scan of two copolymers of vinyl alcohol with methyl acrylate which can be used in this invention. Crystalline PVA/MA 9% copolymer is a crystalline vinyl alcohol copolymer containing 9 percent methyl acrylate. Amorphous PVA/MA 6% copolymer is an vinyl alcohol copolymer with 6% methyl acrylate.

FIG. 2 is a plot of phenylpropanolamine (PPA) concentration in plasma of dogs administered the drug (PPA) intravenously, orally by capsule and orally in an amorphous PVA/MA 6% copolymer tablet of this invention, and shows the prolonged release of drug by the tablet.

FIG. 3 is a plot showing dissolution profile of theophylline from PVA tablets prepared as described in Examples 1-4.

FIG. 4 is a plot showing dissolution profile of phenylpropanolamine (PPA) from PVA tablets prepared as described in Example 5.

Fig. 5 is a plot showing dissolution profile of potassium monopersulfate from PVA tablets prepared according to Example 6.

## DETAILED DESCRIPTION

As a general comment on the nomenclature used in connection with the rate of release of active ingredient from the tablets of the present invention, there is lack of uniformity between medical applications on the one hand and agricultural and/or industrial applications on the other hand. Accordingly, the rate of release has been arbitrarily classified for the purpose of the present disclosure and claims into the following rough categories: instant (a few seconds to a few minutes), immediate (30 minutes), short (90% released in 4 hours), intermediate (85-90% released in 1-2 days), and extended sustained (90% released in 4 days).

Binders

Commercially available polyvinyl alcohols which have the physical and chemical characteristics required for this invention are the Elvanol® polyvinyl alcohol homopolymers, which are manufactured and sold by E. I. du Pont de Nemours and Company. These crystalline polymers have viscosities of 10-70 mPa.s; number average molecular weight $\overline{M}n$ of 25,000-100,000; polydispersity of 1.4 to 2.6; surface area of 0.6-6.0; percent hydrolysis of 99.0-99.8; and a particle size distribution such that less than 5% is retained on a 20 mesh (0.833 mm) screen, less than 50% is retained on an 80 mesh (0.175 mm) screen, more than 70% is retained on a 200 mesh (0.074 mm) screen, and more than 90% is retained on a 400 mesh (0.038 mm) screen. In a photomicrograph, the polymers have the appearance of spheroidal clusters of spheroidal particles. The particles are capable of flowing spontaneously through an orifice of 10 mm diameter and are compressible as pure material to a tablet having a hardness greater than 30 SCU with a compression pressure of 12.3 x $10^6$ kg/m$^2$ (12.46 x $10^7$ Pa).

A preferred Elvanol® PVA is Elvanol® HV, which is referred to below as crystalline PVA-homopolymer. This polymer has a viscosity of 55-65 mPa.sec.

Crystalline vinyl alcohol/methyl acrylate copolymers for use in this invention can be prepared in a continuous polymerizer at 70-103 kPa from vinyl acetate monomer (VA) and methyl acrylate (MA) mixtures. The VA/MA mixture is purged with nitrogen to remove any residual air, and is mixed with methanol, recycled solvents and Vazo® 64 2,2'-azobis(isobutyronitrile), the initiator for the reaction. This mixture is continuously fed to a stirred polymerizer kettle maintained under reflux. A solution containing vinyl acetate/methyl acrylate copolymer, methanol, methyl acetate and VA/MA is removed continuously and polymerization is inhibited by addition of hydrazine monoacetate. Then, the solution is fed to the top of a stripper column. Methanol vapors at atmospheric pressure enter the bottom of the column and strip the VA/MA from the polymer. The VA/MA and methanol vapors are removed from the top of the column and a solution containing vinyl acetate/methyl acrylate copolymer, methanol and methyl acetate is removed from the bottom.

The copolymer/methanol/methyl acetate solution is pulse-fed to a stirred alcoholysis kettle maintained at a constant temperature of 60-65°C. As the vinyl acetate copolymer is converted to vinyl alcohol copolymer, methyl acetate is produced. The vinyl alcohol copolymer is insoluble in the methanol/methyl acetate and the polymer precipitates. The slurry overflows to a holding tank and is neutralized to pH 5-7 by addition of acetic acid.

The neutralized slurry is then heated to a temperature of 110-140°C and held for 5-20 minutes at this temperature. This heat treatment improves the cold water slurrying characteristics of the copolymer, and causes crystallization in at least the surface portion of the PVA particles. The slurry is centrifuged and the cake is dried in a steam tube rotary dryer. Solvents and monomer are recovered and recycled.

The solution/slurry alcoholysis step described above is key to obtaining granular PVA having greater than 98% hydrolysis and the other physical characteristics required for use in this invention. In other commercial PVA manufacturing processes the polyvinyl acetate (PVAc) solution is run out onto a belt and the belt is run through an alkaline methanol solution to convert the polymer to PVA. This alcoholysis method results in relatively low percent hydrolysis and produces non-spheroidal PVA particles with poor flow and compression characteristics.

The use of pulse feed alcoholysis to produce granular PVA is disclosed in Tanner U.S. Patent 3,296,236, issued January 3, 1967. Heat treatment to improve water slurrying properties is disclosed in Bristol U.S. Patent 3,654,247, issued April 4, 1972, and Bristol U.S. Patent 3,497,487, issued February 24, 1970. Stripping VA monomer is disclosed in Lankton et al. U.S. Patent 3,259,555, issued July 5, 1966. Use of hydrazine to inhibit vinyl polymerization is disclosed in Bristol et al. U.S. Patent 3,583,963, issued June 8, 1971. The complete disclosures of these patents are incorporated herein by reference.

A PVA copolymer containing 9% methyl acrylate has been prepared by the above-described procedure. This product is referred to herein as crystalling PVA/MA 9% copolymer. Crystalline vinyl alcohol/methyl methacrylate copolymers for use in this invention can be made by the same procedure, substituting methyl methacrylate for methyl acrylate. The methyl acrylate an methyl methacrylate are present in the vinyl alcohol copolymer as lactones. For the PVA copolymers, the percentage refers to the grams of comonomer (MA or MMA) per 100 grams of polymer, assuming the original molecular weight of the comonomer.

A morphous PVA homopolymer and copolymers with methyl acrylate and methyl methacrylate can be prepared by the above-described procedure, except eliminating the heat treatment step. However, for making the amorphous polymers used in the examples a modified procedure was used. The modified polymerization procedure uses sodium nitrite instead of hydrazine monoacetate to stop the polymerization and uses citric acid to inhibit ester exchange reactions. Stripping of the polymerizer kettle bottoms is accomplished in a vacuum oven overnight at 75°C to remove the VA monomer, methanol and methyl acetate. The polymerizer kettle is operated continuously at 83 kPa. As the PVAc solution is removed from the kettle, polymerization is inhibited by addition of sodium nitrite, then the polymer solution is stripped as described above.

The modified alcoholysis procedure is batch, rather than semi-continuous (pulse-fed) as in the previously-described procedure. The alcoholysis begins by dissolving the polymer obtained as just described in methanol. A solution containing 50 g of polymer, 300 ml of methanol, and 75 ml of methyl acetate is poured into a high-speed, explosion-proof blender. Seventy-five ml of 10% (in methanol) sodium methylate is slowly added to the mixture as it is blended. Temperature is not controlled in the alcoholysis kettle; the unit starts out at ambient temperature and temperature rises due to reaction and mixing. After a gel phase has formed and been broken, the mixture is blended for 10 more minutes. The mixture is then neutralized to pH 5-7 with acetic acid. The cake is then filtered, washed with methanol, filtered again, screened through a 20 mesh (0.833 mm) screen, and dried in a vacuum oven at 70°C. The polymer is not heat-treated.

PVA homopolymer made by this modified procedure is referred to herein as amorphous PVA homopolymer. A PVA copolymer containing 6% methyl acrylate made by this modified procedure is referred to herein as amorphous PVA/MA 6% copolymer. The methyl acrylate is present in the final product as a lactone.

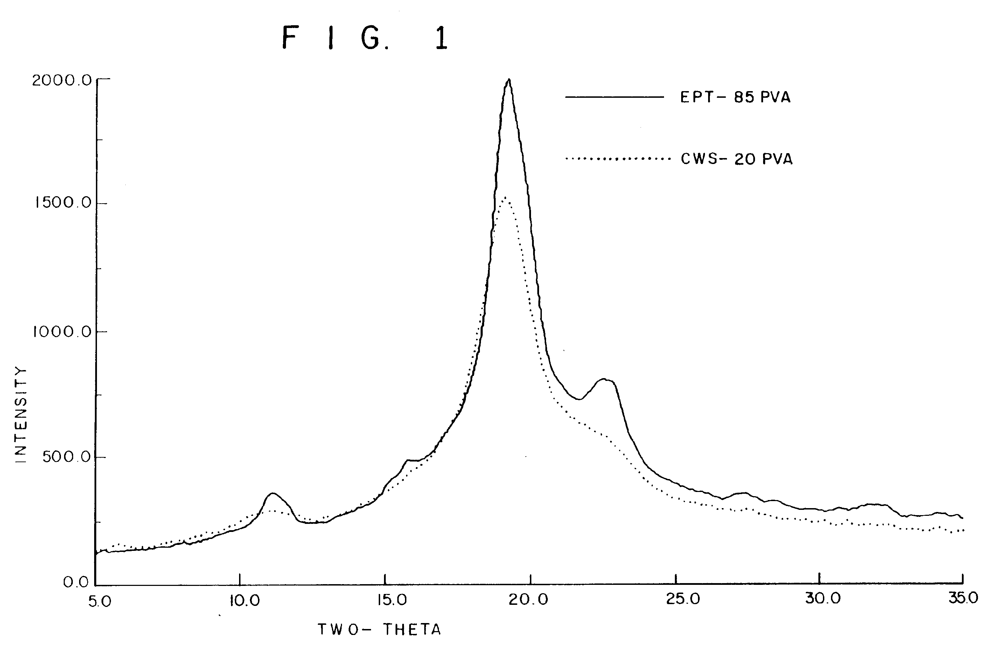

Figure 1 is a wide angle x-ray diffraction scan for crystalline PVA/MA 9% copolymer and amorphous PVA/MA 6% copolymer. A polymer is considered amorphous if an X-ray diffraction scan of the polymer is characterized by the lack of distinct peaks in the angular region of the scan dominated by chain-chain interactions. These crystalline peaks are the equatorial reflections (Miller indices hko for a polymer where the polymer-chain axis is coincident with the c-axis of the unit cell). More specifically, the lack of distinct peaks in the region between 13 degrees and 35 degrees 2-theta can be tested by the lack of any distinct minima in this region other than the low- and high-angle limits of the broad amorphous peak. A distinct minimum is characterized by having a slope of the first derivative of zero where the curvature or the second derivative is positive or concave upwards. A scan for this test can be obtained on any well-aligned reflection powder diffractometer employing a nickel filter or monochromating crystal and pulse-height analysis set to pass symmetrically 90% of the characteristic copper radiation.

Referring to Fig. 1, it will be observed that amorphous PVA/MA 6% copolymer produced no distinct peak in the angular region dominated by chain-chain interactions, i.e., the region of 15-35 degrees 2-theta, other than the region 19 degrees 2-theta which corresponds to the amorphous peak. A shoulder in the 23-degree 2-theta region suggests some chain-chain interaction, but the polymer is considered to be substantially amorphous. Crystalline PVA/MA 9% copolymer, on the other hand produced, a distinct peak at 23 degrees 2-theta, indicating that it is at least partially crystalline. It is believed that the particles of crystalline PVA/MA 9% copolymer have a crystalline surface and amorphous core.

X-ray diffraction scans for crystalline PVA homopolymer and amorphous PVA homopolymer are similar to those for the crystalline and amorphous copolymers, respectively.

A crystalline granular polymer can be converted to a substantially amorphous granular polymer by dissolving in a methanol/acetone mixture and reprecipitating by addition of methanol without heat treatment. Similarly, a substantially amorphous polymer can be converted to a crystalline polymer by slurrying in methanol and heat treating under conditions similar to the heat treatment described above.

It is possible, though not preferred, to use conventional binders, such as those mentioned in the Background of the Invention section, in combination with the vinyl alcohol polymers described above. For example, the vinyl alcohol polymer could constitute 30-100% of total binder and 0-70% could be one or more of the conventional binders or a non-granular vinyl alcohol polymer.

Tableting

For preparation of tablets binder and active ingredient substance are mixed in conventional manner, using conventional equipment. The mixture can be wet granulated in the conventional way, but dry granulation is preferred and direct compression is most preferred.

The active ingredient can be any active ingredient or mixture of active ingredients capable of being administered or employed in tablet form. We have formulated oxycodone, nalbuphine, phenylpropanolamine, and theophylline as PVA tablets. Examples of other active ingredients which can be used are those drugs and drug classes listed in Schor et al. U.S. Patent 4,389,393, issued June 21, 1983, the disclosure of which is incorporated herein by reference.

The ratio of active ingredient to binder will generally be in the range of 3:1 to 1:10, preferably 2:1 to 1:5,

EP 0 359 746 B1

most preferably 1:1 to 1:3.

In addition to binder and active ingredient, other commonly used tablet fillers and excipients can be used in conventional amounts. Inclusion of magnesium stearate as a lubricant in an amount of up to about 1% of the total ingredients is preferred. Other excipients which can be used include other lubricants, flavoring agents, disintegrants, and coloring agents.

Any conventional tableting machine can be used, and tablets can be made in any conventional size and shape, e.g., discoid, oblong, or triangular. Compression pressures up to the maximum provided by the machine can be used, e.g., $1.6 \times 10^8$ kg/m$^2$ ($16.2 \times 10^8$ Pa) or more, but a pressure in the range of $2\text{-}5 \times 10^6$ kg/m$^2$ ($20.3 - 50.7 \times 10^6$ Pa) will usually be sufficient and preferred for economy.

EXAMPLES

In Examples 1-4, below, the drug release characteristics were measured in vitro. Dissolution was performed using a procedure described in the U.S. Pharmacopeia XXI, page 1243 (1985). This procedure involves use of a 1 liter glass vessel immersed in water at 30°C or 37°C and an appropriate dissolution medium (0.1 N HCl, pH 7.4 phosphate buffer, buffered saline or water). This vessel is stirred at a constant rate (25, 50 or 100 RPM) for the duration of the dissolution procedure to determine the amount of drug released. In Examples 1 to 4, 900 ml of distilled water was used set at 37°C with a stirring speed of 50 RPM.

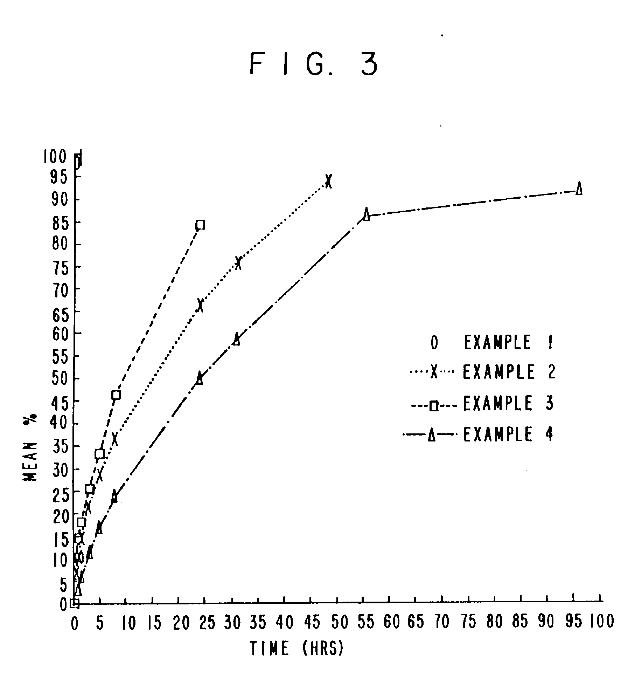

The dissolution profile for Examples 1 to 4 are shown in FIG. 3.

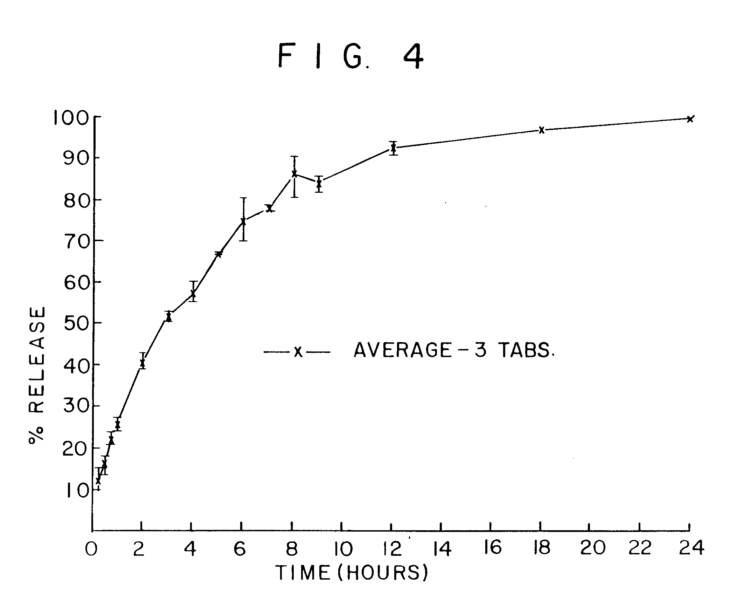

In Example 5, drug release was measured in vitro by the same procedure, where the dissolution medium contained 1000 ml of 0.1 N HCl and was stirred at 50 RPM. The dissolution profile is shown in FIG. 4.

EXAMPLE 1

### Immediate Release (100% Release in 30 Minutes) Theophylline Formula

| Ingredient | mg/Tablet |
|---|---|
| Theophylline | 200 |
| Crystalline PVA homopolymer | 200 |
| Magnesium Stearate | 5 |
| | 405 |

Compounding

1. Blend via geometric dilution.
2. Compress on Manesty F-3 single punch tablet press at $2.7 \times 10^6$ kg/m$^2$ ($2.74 \times 10^7$ Pa) pressure with 3/8 inch (9.53 mm) diameter standard concave tooling to form tablets with average hardness of 12 SCU.

EXAMPLE 2

Intermediate Release Formula (90% Release in 48 Hours)

Same formula and procedure as Example 1 except substitution of non-heat treated amorphous PVA homopolymer. Average hardness of resulting tablets 12 SCU.

7

EXAMPLE 3

### Intermediate Release Formula
### (85% Release in 24 Hours)

| Ingredient | mg/Tablet |
|---|---|
| Theophylline | 200 |
| Crystalline PVA homopolymer | 100 |
| Amorphous PVA/MA 6% copolymer | 100 |
| Magnesium Stearate | 5 |
| | 405 Compounding |

1. Blend via geometric dilution
2. Slug to improve blend flowability - grind to acceptable tablet granulation.
3. Compress on Manesty F-3 tablet press at 2.7 x $10^6$ Kg/m$^2$ (2.74 x $10^7$ Pa) pressure with 9.53 mm diameter standard concave tooling to make tablets with a hardness of 12 SCU.

EXAMPLE 4

### Extended Sustained Release
### (90% Release in 96 Hours)

| Ingredient | mg/Tablet |
|---|---|
| Theophylline | 200 |
| Amorphous PVA/MA 6% copolymer | 200 |
| Magnesium Stearate | 5 |
| | 405 |

Same method of compounding as Example 3. Average hardness of resulting tablets 12 SCU.

EXAMPLE 5

### Phenylpropanolamine (PPA) Sustained Release Tablet
### (In Vitro - 100% Release in 24 Hours)

| Ingredient | mg/Tablet |
|---|---|
| Amorphous PVA/MA 6% copolymer | 237 |
| Phenylpropanolamine (PPA) | 60 |
| Magnesium Stearate | 3 |
| | 300 |

Compounding

1. Blend via geometric dilution.
2. Tablet on Manesty F-3 single punch tableting machine with 3/8 inch (9.53 cm) diameter standard concave tooling and compression pressure of 4.8 x $10^6$ kg/m$^2$ (4.86 x $10^7$ Pa) to form compacts at the target weight of 300 mg, tablet hardness = 12 SCU.

Three dogs were administered, in a cross-over fashion, phenylpropanolamine.HCl i.v. (3 mg/kg), orally (30 mg in a gelatin capsule) and in the above PVA/MA copolymer tablet composition containing 60 mg of phenylpropanolamine.HCl. Plasma samples were collected as a function of time and frozen until analysis of drug concentration.

Analysis of phenylpropanolamine in plasma was performed by high pressure liquid chromatography (HPLC). Phenylethylamine, as internal standard, and 3.5% aqueous sodium carbonate were added to 0.5 ml plasma samples. Two extractions into ethyl acetate and back extraction into 0.2 ml of 5% aqueous acetic acid were performed. The acetic acid solution was injected onto the HPLC. The mobile phase consisted of a mixture of 11% (v/v) acetonitrile and 0.21 (v/v) 1N.HCl in 0.004 M aqueous sodium heptane sulfonate. A 25 cm CN column and U.V. detection at = 210 nm were employed. Phenylpropanolamine/phenylethylamine peak area ratios were used in construction of calibration curves.

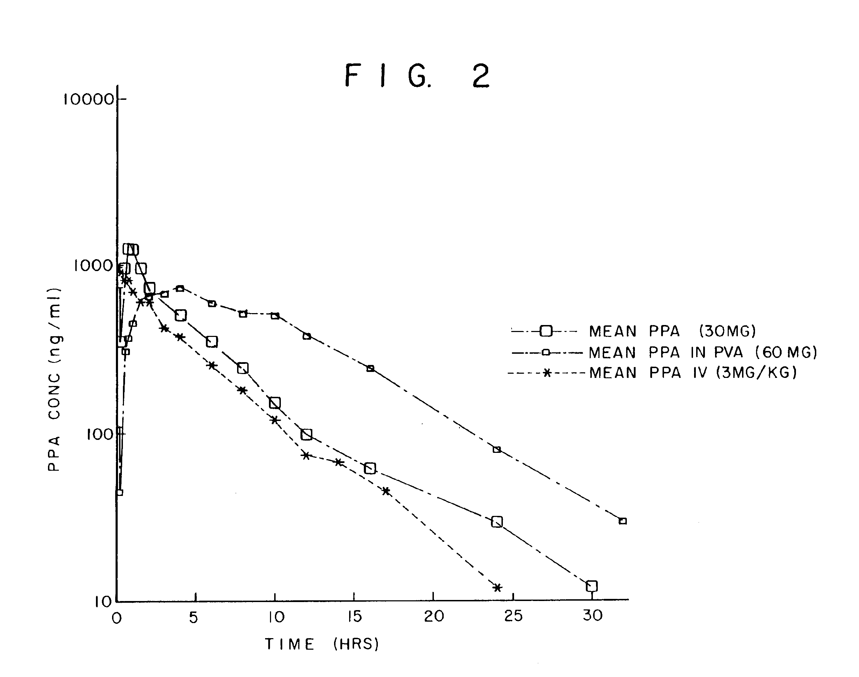

The area under each plasma phenylpropanolamine concentration vs. time curve (AUC) (Fig. 2) was calculated using the trapezoidal method. Bioavailability (F) was estimated by:

$$F = \frac{AUC(po) \times Dose\ (iv)}{SUC(iv) \times Dose\ (po)} \times 100$$

F represents the percentage of the administered dose absorbed into plasma.

Sustained plasma concentrations of the drug were observed for 12 hours (Fig. 2), i.e., a relatively constant plasma concentration is achieved.

The oral bioavailability of phenylpropanolamine.HCl when administered in the amorphous PVA/MA 6% copolymer formulation was 78 + 4% (mean + SD in 3 dogs) and that of phenylpropanolamine.HCl when administered in a gelatin capsule was 98 + 8%.

## EXAMPLE 6

Tablets containing 50% of potassium monopersulfate (commercial oxidizing agent available from the Du Pont Company under the trademark Oxone®) and 50% of an amorphous PVA/MA 9% copolymer were prepared according to the technique described in Example 1. The rate of release of the active ingredient is shown in Fig. 5, which is a plot of percent release versus time in hours. It can be seen that 90% of the active ingredient was released in 4 hours, where a plateau was reached. These can be characterized as short release tablets.

When comparing these results with those of Example 4, wherein a fairly similar PVA/MA copolymer was used in the same proportion as the tableting material, one notes a considerable difference in the respective rates of release. This may be due to the difference in the properties of the respective active ingredients. While both theophylline and potassium persulfate have similar water solubilities, the persulfate is an energetic oxidizing agent and tends to release gas on contact with water and with organic copolymer.

## EXAMPLE 7

A commercial herbicide material (metasulfuron methyl, available from the Du Pont Company) was compounded according to the technique of Example 1 as follows:

```
metasulfuron methyl                70 parts
Crystalline PVA/MA 9% copolymer 30 parts
```

When added to a low electrolyte strength nitrogen fertilizer (28% N, 0% K, 0% P), the active ingredient was completely released in 3 minutes; when added to high electrolyte strength nitrogen fertilizer (32% N, 0% K, 0% P), it was completely released in 7 minutes. These were instant release tablets, suitable for use in fertilizer spray solutions.

## EXAMPLE 8

Hexazinone herbicide was compounded according to the technique of Example 1 as follows:

| | |
|---|---|
| Hexazinone | 20 parts |
| Crystalline EVA hompolymer | 80 parts |

The rate of release of the active ingredient in water was 30 seconds; thus the release was instant. These tablets are suitable for use in aerial broadcasting, where it is not necessary to dust the plants but it is sufficient to deliver the systemic herbicide to the roots, as it dissolves in the moisture of the soil.

EXAMPLE 9

A slug bait could be compounded as follows:

wheat middlings (and poison)          60 parts

Amorphous PVA/MA 9% copolymer 40 parts

In a test in which no poison was used, disintegration of tablets in water occurred in 30 min. The pellets were palatable to slugs. By contrast, standard prior art molluscicide tablets are formulated with sugar. Under the same accelarated laboratory conditions, such sugar-formulated tablets disintegrate in 20 minutes. Under field conditions, tablets of the present invention are only slowly rained away, while the prior art tablets are rained away considerably faster. It is estimated that the tablets of the present invention (when formulated with active ingredient, such as, for example metaldehyde) will have sufficiently long sustained activity to last for a full season, while the prior art tablets normally must be applied twice during the same period.

All the above examples show the advantages of using the polymers of the present invention as tableting materials. However, the actual rates of release of the active ingredient present in such tablets will depend to some extent not only on the polymer itself but also on the nature of the active ingredient, including its water solubility and activity or chemical reactivity.

**Claims**

1. A method of making a compressed tablet for delivery of an active ingredient comprising mixing active ingredient and binder in powder form and compressed the mixture into a tablet, characterized by that the binder consists essentially of at least one polymer selected from the group consisting of polyvinyl alcohol and copolymers of vinyl alcohol with up to 10 mole percent methyl acrylate or methyl methacrylate, the polymer having: a number average moleculer weight in the range of 25,000 to 100,000; polydispersity in the range of 1.4 to 2.6; viscosity in 4% aqueous solution in the range of 10 to 70 mPa.s; a surface area in the range of 0.6 to 6.0 $m^2$/g; percent hydrolysis of at least 98%; the polymer being in the form of spheroidal particles arranged in spheroidal clusters, with a size distribution such that less than 5% of the polymer is retained on a 20 mesh (0.833 mm) screen, less than 50% is retained on an 80 mesh (0.175 mm) screen, and more than 70% is retained on a 200 mesh (0.074 mm) screen.

2. Method of claim 1 wherein the polymer is capable of flowing spontaneously through an orifice of 10 millimeter diameter and is compressible to a tablet having a hardness greater than 30 Strong-Cobb units with a compression pressure of $12.3 \times 10^6$ kg/m2 ($12.46 \times 10^7$ Pa).

3. Method of claim 2 wherein the binder consists essentially of at least one polymer selected from the group consisting of polyvinyl alcohol and copolymers of vinyl alcohol with up to about 10 mole percent methyl acrylate.

4. Method of claim 3 wherein the binder consists essentially of amorphous polymer, whereby the resulting table provides prolonged active ingredient release.

5. Method of claim 3 wherein the binder consists essentially of at least partially crystalline polymer, whereby the resulting tablet provides immediate active ingredient release.

6. Method of claim 3 wherein the binder consists essentially of a mixture of substantially amorphous polymer and at least partially crystalline polymer, whereby the resulting tablet provides intermediate active ingredient release.

7. Method of claim 3 wherein the mixture is dry granulated prior to compression to tablet form.

8. Method of claim 3 wherein the tablet is formed by direct compression of the mixture without prior granulation.

9. Compressed tablet for active ingredient delivery characterized in that it consists essentially of active ingredient and binder consisting essentially of 30-100% of polymer selected from the group consisting of copolymers of vinyl alcohol with 1 to 10 mole percent methyl acrylate or methyl methacrylate, the polymer having a molecular weight in the range of 25,000 to 100,000; polydispersity in the range of 1.4 to 2.6; viscosity in 4% aqueous solution in the range of 10 to 70 mPa.s; a surface area in the range of 0.6 to 6.0 $m^2$/g; percent hydrolysis of at least 98%; the polymer being in the form of spheroidal particles arranged in spheroidal clusters, with a size distribution such that less than 5% of the polymer is retained on a 20 mesh (0.833 mm) screen, less than 50% is retained on an 80 mesh (0.175 mm) screen, and more than 70% is retained on a 200 mesh (0.074 mm) screen and 0 to 70% of at least one member of the group consisting of polyvinyl alcohol, hydroxypropylmethylcellulose, microcrystalline cellulose, lactose, acacia mucilage, tragacanth mucilage, starch mucilage, alginates, sugar, and polyvinyl pyrrolidone.

10. Compressed tablet of claim 9 wherein the active ingredient is a drug to be administered orally.

EP 0 359 746 B1

11. Compressed tablet of claim 9 wherein the binder consists essentially of 30-100% of vinyl alcohol copolymer with 1 to 10 mole percent methyl acrylate and 0-70 percent granular, crystalline polyvinyl alcohol.

12. Compressed tablet of claim 11 wherein the active ingredient is a drug to be administered orally.

13. Compressed tablet of claim 11 wherein the active ingredient is an agricultural chemical.

14. Compressed tablet of claim 11 wherein the active ingredient is a veterinary chemical.

15. Compressed tablet of claim 11 wherein the active ingredient is a personal care chemical.

16. Compressed tablet of claim 11 wherein the vinyl alcohol copolymer is amorphous, whereby the tablet provides prolonged active ingredient release.

17. Compressed tablet of claim 16 wherein the binder consists essentially of amorphous copolymer.

18. Compressed tablet for immediate oral drug delivery consisting essentially of drug substance and binder characterized in that the binder consists essentially of at least one polymer selected from the group consisting of polyvinyl alcohol and copolymers of vinyl alcohol with up to 10 mole percent of methyl acrylate or methyl methacrylate, the polymer having: a number average molecular weight in the range of 25,000 to 100,000; polydispersity in the range of 1.4 to 2.6; viscosity in 4% aqueous solution in the range of 10 to 70 mPa.s; a surface area in the range of 0.6 to 6,0 $m^2$/g; percent hydrolysis of at least 98%; the polymer being in the form of spheroidal particles arranged in spheroidal clusters, with a size distribution such that less than 5% of the polymer is retained on a 20 mesh (0.833 mm) screen, less than 50% is retained on a 80 mesh (0.175 mm) screen, and more than 70% is retained on a 200 mesh (0.074 mm) screen, said polymer is being at least partially crystalline, whereby the tablet disintegrates quickly when placed in dilute acid.

19. Compressed tablet of claim 18 wherein the polymer is polyvinyl alcohol.

20. Compressed tablet of claim 18 wherein the polymer is a copolymer of vinyl alcohol and methyl acrylate.

21. Compressed tablet of claim 18 wherein the active compound is wet granulated with an aqueous solution ranging in concentration from 1 to 10% polyvinyl alcohol homopolymer and/or copolymer.

**Patentansprüche**

1. Verfahren zur Herstellung einer gepreßten Tablette zur Abgabe eines Wirkstoffs durch Mischen von Wirkstoff und Bindemittel in Pulverform und Pressen der Mischung in eine Tablette, dadurch gekennzeichnet, daß das Bindemittel im wesentlichen aus wenigstens einem aus der aus Polyvinylalkohol und Copolymeren von Vinylalkohol mit bis zu 10 Mol-% Methylacrylat oder Methylmethacrylat bestehenden Gruppe ausgewählten Polymer besteht, wobei das Polymer ein Zahlenmittel-Molekulargewicht im Bereich von 25 000 bis 100 000, eine Polydispersität im Bereich von 1,4 bis 2,6, eine Viskosität in 4 %iger wässriger Lösung im Bereich von 10 bis 70 mPa.s, eine Oberfläche im Bereich von 0,6 bis 6,0 $m^2$/g und eine prozentuale Hydrolyse von wenigstens 98 % aufweist und das Polymer in Form von in sphäroidalen Clustern angeordreten sphäroidalen Teilchen mit einer Größenverteilung vorliegt, daß weniger als 5 % des Polymers auf einem Sieb von 20 mesh (0,833 mm) zurückgehalten werden, weniger als 50 % auf einem Sieb von 80 mesh (0,175 mm) zurückgehalten werden und mehr als 70 % auf einem Sieb von 200 mesh (0,074 mm) zurückgehalten werden.

2. Verfahren nach Anspruch 1, worin das Polymer in der Lage ist, spontan durch eine Öffnung von 10 mm Durchmesser zu fließen und mit einem Kompressionsdruck von 12,3 x $10^6$ kg/$m^2$ (12,46 x $10^7$ Pa) in eine Tablette mit einer Härte von mehr als 30 Strong-Cobb-Einheiten kompressibel ist.

3. Verfahren nach Anspruch 2, worin das Bindemittel im wesentlichen aus wenigstens einem aus der aus Polyvinylalkohol und Copolymeren von Vinylalkohol mit bis zu etwa 10 Mol-% Methylacrylat bestehenden Gruppe ausgewählten Polymer besteht.

4. Verfahren nach Anspruch 3, worin das Bindemittel im wesentlichen aus amorphem Polymer besteht, wodurch die resultierende Tablette eine verlängerte Wirkstofffreisetzung ergibt.

5. Verfahren nach Anspruch 3, worin das Bindemittel im wesentlichen aus wenigstens teilweise kristalinem Polymer besteht, wodurch die resultierende Tablette eine sofortige Wirkstofffreisetzung ergibt.

6. Verfahren nach Anspruch 3, worin das Bindemittel im wesentlichen aus einer Mischung von im wesentlichem amorphem Polymer und wenigstens teilweise kristallinem Polymer besteht, wodurch die resultierende Tablette eine mittlere Wirkstofffreisetzung ergibt.

7. Verfahren nach Anspruch 3, worin die Mischung vor der Pressung in Tablettenform trocken granuliert wird.

8. Verfahren nach Anspruch 3, worin die Tablette durch direktes Pressen der Mischung ohne vorherige Granulierung gebildet wird.

9. Gepreßte Tablette für die Wirkstoffabsabe, dadurch gekennzeichnet, daß sie im wesentlichen aus Wirkstoff und Bindemittel besteht, das im wesentlichen aus 30 bis 100 % aus der aus Copolymeren von Vinylalkohol mit 1 bis 10 Mol-% Methylacrylat oder Methylmethacrylat bestehenden Gruppe ausgewähltem Polymer

11

besteht, wobei das Polymer ein Molekulargewicht im Bereich von 25 000 bis 100 000, eine Polydispersität im Bereich von 1,4 bis 2,6, eine Viskosität in 4 %iger wässriger Lösung im Bereich von 10 bis 70 mPa.s, eine Oberfläche im Bereich von 0,6 bis 6,0 m²/g und eine prozentuale Hydrolyse von wenigstens 98 % aufweist und das Polymer in Form von in sphäroidalen Clustern angeordneten sphäroidalen Teilchen in einer Größenverteilung vorliegt, daß weniser als 5 % des Polymers auf einem Sieb von 20 mesh (0,833 mm) zurückgehalten werden, weniger als 5 % auf einem Sieb von 80 mesh (0,175 mm) zurückgehalten werden und mehr als 70 % auf einem Sieb von 200 mesh (0,074 mm) zurückgehalten werden sowie aus wenigstens einem Mitglied der aus Polyvinylalkohol, Hydroxypropylmethylcellulose, mikrokristalliner Cellulose, Lactose, Akazienschleim, Tragacanthschleim, Stärkenschleim, Alginaten,Zucker und Polyvinylpyrrolidon bestehenden Gruppe.

10. Gepreßte Tablette nach Anspruch 9, worin der Wirkstoff ein oral zu verabreichendes Medikament ist.

11. Gepreßte Tablette nach Anspruch 9, worin das Bindemittel im wesentlichen aus 30 bis 100 % Vinylalkoholcopolymer mit 1 bis 10 Mol-% Methylacrylat und 0 bis 70 % granularem kritallinem Polyvinylalkohol besteht.

12. Gepreßte Tablette nach Anspruch 11, worin der Wirkstoff ein oral zu verabreichendes Medikament ist.

13. Gepreßte Tablette nach Anspruch 11, worin der Wirkstoff eine landwirtschaftliche Chemikalie ist.

14. Gepreßte Tablette nach Anspruch 11, worin der Wirkstoff eine veterinäre Chemikalie ist.

15. Gepreßte Tablette nach Anspruch 11, worin der Wirkstoff eine Chemikalie für die persönliche Pflege ist.

16. Gepreßte Tablette nach Anspruch 11, worin das Vinylalkoholcopolymer amorph ist, wodurch die Tablette eine verlängerte Wirkstofffreisetzung ergibt.

17. Gepreßte Tablette nach Anspruch 16, worin das Bindemittel im wesentlichen aus amorphem Copolymer besteht.

18. Gepreßte Tablette für die sofortige orale Medikamentfreisetzung, welche im wesentlichen aus Medikamentsubstanz und Bindemittel besteht, dadurch gekennzeichnet, daß das Bindemittel im wesentlichen aus wenigstens einem aus der aus Polyvinylalkohol und Copolymeren von Vinylalkohol mit bis zu 10 Mol-% Methylacrylat oder Methylmethacrylat bestehenden Gruppe ausgewählten Polymer besteht, wobei das Polymer ein Zahlenmittel-Molekulargewicht im Bereich von 25 000 bis 100 000, eine Polydispersität im Bereich von 1,4 bis 2,6, eine Viskosität in 4 %iger wässriger Lösung im Bereich von 10 bis 70 mPa.s, eine Oberfläche im Bereich von 0,6 bis 6,0 m²/g und eine prozentuale Hydrolyse von wenigstens 98 % aufweist und das Polymer in Form von in sphäroidalen Clustern angeordneten sphäroidalen Teilchen mit einer Größenverteilumg vorliegt, daß weniger als 5 % der Polymers auf einem Sieb von 20 mesh (0.833 mm) zurückgehalten werden, weniger als 50 % auf einem Sieb von 80 mesh (0,175 mm) zurückgehalten werden und mehr als 70 % auf einem Sieb von 200 mesh (0,074 mm) zurückgehalten werden, und wobei das Polymer wenigstens teilweise kristallin ist, wodurch die Tablette sich schnell zersetzt, wenn in verdünnte Säure gegeben.

19. Gepreßte Tablette nach Anspruch 18, worin das Polymer Polyvinylalkohol ist.

20. Gepreßte Tablette Anspruch 18, worin das Polymer ein Copolymer von Vinylalkohol und Methylacrylat ist.

21. Gepreßte Tablette nach Anspruch 18, worin der Wirkstoff mit einer wässrigen Lösung mit einer Konzentration im Bereich von 1 bis 10 % Polyvinylalkoholhomopolymer und/oder -copolymer naß granuliert ist.

**Revendications**

1. Un procédé de confection d'une pastille comprimée destinée à la distribution d'un ingrédient actif consistant à mélanger un ingrédient actif et un liant sous forme de poudre et à comprimer le mélange en pastilles, caractérisé en ce que le liant est essentiellement constitué d'au moins un polymère choisi dans le groupe formé par l'alcool polyvinylique et les copolymères d'alcool vinylique avec jusqu'à 10% en moles de méthylacrylate ou de méthylméthacrylate, le polymère ayant un poids moléculaire moyen en nombre compris entre 25 000 et 100 000, une polydispersité comprise entre 1,4 et 2,6; une viscosité dans une solution aqueuse à 4% comprise entre 10 et 70 mPa.s; une surface spécifique comprise entre 0,6 et 6,0 m²/g; un pourcentage d'hydrolyse d'au moins 98%; le polymère se présentant sous la forme de particules sphéroïdales rassemblées en grappes sphéroïdales, sa distribution de dimension étant telle que moins de 5% du polymère sont retenus sur un tamis de 20 mailles (0,833 mm); moins de 50% sont retenus sur un tamis de 80 mailles (0,175 mm); et plus de 70% sont retenus sur un tamis de 200 mailles (0,074 mm).

2. Procédé selon la revendication 1, caractérisé en ce que le polymère est capable de s'écouler spontanément par un orifice de 10 mm de diamètre et qu'il est compressible en une pastille présentant une dureté supérieure à 30 unités Strong-Cobb avec une pression de compression de $12,3 \times 10^6$ g/m² ($12,46 \times 10^7$ Pa).

3. Procédé selon la revendication 2, caractérisé en ce que le liant est essentiellement constitué d'au moins un polymère choisi dans le groupe formé par l'alcool polyvinylique et les copolymères d'alcool vinylique avec jusqu'à environ 10% en moles de méthylacrylate.

4. Procédé selon la revendication 3, caractérisé en ce que le liant est essentiellement constitué d'au moins un polymère amorphe, les pastilles obtenues procurant une libération prolongée de l'ingrédient actif.

5. Procédé selon la revendication 1, caractérisé en ce que le liant est essentiellement constitué d'au moins un polymère au moins partiellement cristallin, les pastilles ainsi obtenues procurant une libération immédiate de l'ingrédient actif.

6. Procédé selon la revendication 3, caractérisé en ce que le liant est constitué essentiellement d'un mélange de polymères sensiblement amorphes et d'un polymère au moins partiellement cristallin, les pastilles obtenues procurant une libération intermédiaire de l'ingrédient actif.

7. Procédé selon la revendication 3, caractérisé en ce que le mélange est granulé à sec avant la compression en forme de pastilles.

8. Procédé selon la revendication 3, caractérisé en ce que la pastille est moulée par compression directe du mélange sans granulation préalable.

9. Pastille comprimée pour distribution d'ingrédients actifs, caractérisée en ce qu'elle est essentiellement constituée d'ingrédient actif et d'un liant constitué essentiellement de 30 à 100% de polymère choisi dans le groupe formé par des copolymères d'alcool vinylique avec 1 à 10% en moles de méthylacrylate ou méthylméthacrylate, le poids moléculaire moyen du polymère étant compris entre 25 000 et 100 000, sa polydispersité comprise entre 1,4 et 2,6; sa viscosité dans une solution aqueuse à 4% comprise entre 10 et 70 mPa.s; sa surface spécifique comprise entre 0,6 et 6,0 m2/g; son pourcentage d'hydrolyse d'au moins 98%; le polymère se présentant sous la forme de particules sphéroïdales rassemblées en grappes sphéroïdales, sa distribution de dimension étant telle que moins de 5% du polymère sont retenus sur un tamis de 20 mailles (0,833 mm); moins de 50% sont retenus sur un tamis de 80 mailles (0,175 mm); et plus de 70% sont retenus sur un tamis de 200 mailles (0,074 mm) et de 0 à 70% d'au moins un élément du groupe formé par alcool polyvinylique, hydroxypropylméthylcellulose, cellulose microcristalline, lactose, mucilage d'acacia, mucilage d'adranganthe, mucilage d'amidon, alginates, sucre et polyvinylpyrrolidone.

10. Pastille comprimée selon la revendication 9, caractérisée en ce que l'ingrédient actif est un médicament administré par voie orale.

11. Pastille comprimée selon la revendication 9, caractérisée en ce que le liant est constitué essentiellement de 30 à 100% de copolymère d'alcool vinylique avec de 1 à 10 moles% de méthylacrylate et de 0 à 70% d'alcool polyvinylique cristallin granulaire.

12. Pastille comprimée selon la revendication 11, caractérisée en ce que l'ingrédient actif est un médicament administré par voie orale.

13. Pastille comprimée selon la revendication 11, caractérisée en ce que l'ingrédient actif est un produit chimique agricole.

14. Pastille comprimée selon la revendication 11, caractérisée en ce que l'ingrédient actif est un produit chimique vétérinaire.

15. Pastille comprimée selon la revendication 11, caractérisée en ce que l'ingrédient actif est un produit chimique d'hygiène personnelle.

16. Pastille comprimée selon la revendication 11, caractérisée en ce que le copolymère d'alcool vinylique est amorphe, la pastille procurant alors une libération prolongée de l'ingrédient actif.

17. Pastille comprimée selon la revendication 16, caractérisée en ce que le liant est constitué essentiellement d'un copolymère amorphe.

18. Pastille comprimée pour distribution immédiate de médicament par voie orale constituée essentiellement d'une substance médicamenteuse et d'un liant caractérisé en ce que le liant est constitué essentiellement d'au moins un polymère choisi dans le groupe formé par l'alcool polyvinylique des copolymères d'alcool vinylique avec jusqu'à 10% en moles de méthylacrylate et de méthylméthacrylate, le polymère ayant un nombre moléculaire moyen compris entre 25 000 et 100 000, une polydispersité comprise entre 1,4 et 2,6; une viscosité dans une solution aqueuse à 4% comprise entre 10 et 70 mPa.s; une surface spécifique comprise entre 0,6 et 6,0 m2/g; un pourcentage d'hydrolyse d'au moins 98%; le polymère se présentant sous la forme de particules sphéroïdales rassemblées en grappes sphéroïdales, sa distribution de dimension étant telle que moins de 5% du polymère sont retenus sur un tamis de 20 mailles (0,833 mm); moins de 50% sont retenus sur un tamis de 80 mailles (0,175 mm); et plus de 70% sont retenus sur un tamis de 200 mailles (0,074 mm), ce polymère étant au moins partiellement cristallin, de sorte que les pastilles se désintègrent rapidement lorsqu'on les place dans de l'acide dilué.

19. Pastille comprimée selon la revendication 18, caractérisée en ce que le polymère est l'alcool polyvinylique.

20. Pastille comprimée selon la revendication 18, caractérisée en ce que le polymère est un copolymère d'alcool vinylique de méthylacrylate.

21. Pastille comprimée selon la revendication 18, caractérisée en ce que le dérivé actif est granulé à sec en présence d'une solution aqueuse dont les concentrations en homopolymère et/ou copolymère d'alcool polyvinylique sont comprises entre 1 et 10%.

F I G . 1

EPT– 85 PVA
CWS– 20 PVA

TWO–THETA
INTENSITY

# FIG. 2

MEAN PPA (30MG)
MEAN PPA IN PVA (60 MG)
MEAN PPA IV (3MG/KG)

EP 0 359 746 B1

# FIG. 3

Legend:
- O — EXAMPLE 1
- ···X··· — EXAMPLE 2
- ---□--- — EXAMPLE 3
- —△— EXAMPLE 4

Y-axis: MEAN %
X-axis: TIME (HRS)

EP 0 359 746 B1

# F I G. 4

EP 0 359 746 B1